# FASCICULE DE BREVET EUROPEEN

(11) **EP 0 781 550 B1**
(45) Date de publication et mention de la délivrance du brevet: **14.08.2002**
(21) Numéro de dépôt: 96402788.2
(22) Date de dépôt: 18.12.1996
(51) Int. Cl.: A61K 9/70

(54) **Composition pharmaceutique bioadhésive pour la libération contrôlée de principes actifs**
Bioadhäsives Arzneimittel zur kontrollierten Freisetzung von Wirkstoffen
Bioadhesive pharmaceutical composition for the controlled release of active agents

(30) Priorité: 29.12.1995 FR 9515701
(43) Date de publication de la demande: 02.07.1997
(73) Titulaire: LES LABORATOIRES SERVIER, 92200 Neuilly sur Seine (FR)
(72) Inventeur: Rault, Isabelle, 45170 Saint lye la Foret (FR); Pichon, Gérald, 45100 Orleans (FR)

(56) Documents cités:
- EP-A- 0 055 397
- EP-A- 0 376 891
- EP-A- 0 649 650
- US-A- 4 876 092
- DATABASE WPI Week 8850 Derwent Publications Ltd., London, GB; AN 88-356409 XP002014091 & JP 63 265 983 A (SEKISUI CHEM IND KK) , 2 Novembre 1988
- DATABASE WPI Week 9203 Derwent Publications Ltd., London, GB; AN 92-021917 XP002014092 & JP 63 171 564 A (SEKISUI CHEM IND KK) , 15 Juillet 1988

## Description

La présente invention concerne une nouvelle composition pharmaceutique bioadhésive permettant la libération contrôlée de principes actifs de manière locale, dans la cavité buccale ou de manière systématique à travers une muqueuse buccale (jugale ou gingivale), perlinguale, nasale, vaginale ou rectale. La composition pharmaceutique selon l'invention assure une libération plus ou moins rapide du principe actif et peut rester fixée pendant un temps plus ou moins long sur la muqueuse buccale (muqueuse jugale et gencive), perlinguale, nasale, vaginale et rectale.

L'administration par voie transmucosale présente l'avantage au plan métabolique d'éviter la métabolisation importante du principe actif par effet de premier passage hépatique et donc au plan clinique de diminuer les doses administrées en améliorant l'efficacité thérapeutique. Le principe actif ne subit pas les différentes dégradations enzymatiques ou chimiques présentes tout au long du tractus gastro-intestinal, de même que les inconvénients liés au fonctionnement et à la physiologie de l'appareil gastro-intestinal.

Les possibilités d'administration d'un principe actif à travers une muqueuse dépendent de différents facteurs. En particulier, la composition ne doit pas altérer le tissu d'une manière quelconque à la suite d'un contact prolongé et provoquer des irritations, allergies ou sensibilisations et le principe actif doit pouvoir traverser une surface de tissu assez petite à un débit de diffusion suffisant pour l'obtention de taux plasmatiques adaptés aux besoins thérapeutiques.

Une forme bioadhésive présente la propriété d'adhérer à un tissu biologique, par exemple à une muqueuse dans la cavité buccale, et de s'y maintenir pendant un temps plus ou moins long. Le phénomène de bioadhésion est décrit dans la littérature et est assuré par l'établissement de liaison entre un ou des composés de la forme galénique et des groupements chimiques fonctionnels présents à la surface du tissu biologique. Les interactions intervenant dans le mécanisme de bioadhésion sont décrites comme étant de nature physique, mécanique ou chimique. L'Art Antérieur est notamment représenté par le brevet EP-0 376 891.

La composition pharmaceutique selon l'invention, outre le fait qu'elle soit nouvelle, permet d'obtenir un effet bioadhésif intense et une libération contrôlée et reproductible du principe actif.

La composition pharmaceutique bioadhésive de la présente invention se caractérise par l'utilisation :
- d'un polymère (A) consistant en un ou plusieurs copolymères acétate de vinyle/polyvinylpyrrolidone. Cet excipient habituellement utilisé comme liant et désintégrant dans des comprimés, permet de façon surprenante la formulation de films (ou patches) ayant des propriétés bioadhésives intéressantes pour l'administration transmucosale,
- d'un ou de plusieurs principes actifs,
- éventuellement, d'un composé (B) comprenant un ou plusieurs composés comme la cellulose et ses dérivés comme, par exemple, l'éthylcellulose, l'hydroxypropylcellulose, l'hydroxypropylméthyl-cellulose, etc..., les amidons de diverses origines et leurs dérivés, la gomme arabique, la gomme adragante, la gomme guar, la gomme xanthane, le caroube, les carraghénates,
- et, d'excipients jouant le rôle de plastifiants, d'aromatisants, d'édulcorants.

Le composé (A) est constitué d'un ou plusieurs copolymères acétate de vinyle/polyvinylpyrrolidone de formule suivante :

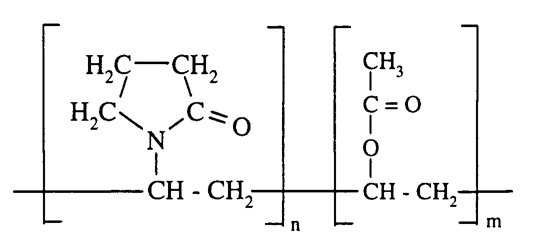

Des exemples précis de ce type de copolymères actuellement disponibles dans le commerce sont le KOLLIDON VA64® (BASF) et les copolymères E-335®, E-535®, E-735®, I-235®, I-335®, I-535®, I-735® et S-630® (ISP).

Le composé (B), quand il est présent dans la forme galénique selon l'invention, permet de prolonger la libération du principe actif.

La bioadhésion et la libération contrôlée sont assurées par le composé (A). La proportion du composé (A) est entre 5 et 85 % en masse. Le type de copolymère acétate de vinyle/polyvinyl-pyrrolidone choisi permet de faire varier la vitesse de solubilisation : plus la proportion de polyvinylpyrrolidone est importante, plus la solubilisation est rapide. La proportion du composé (B), s'il est présent, est entre 5 et 85 % en masse. Selon les proportions utilisées, la libération contrôlée peut être modulée.

D'autre part, les copolymères acétate de vinyle/polyvinylpyrrolidone ont des prix peu élevés, ce qui se traduit par l'obtention de compositions pharmaceutiques à faible coût.

La composition pharmaceutique selon l'invention se présente plus spécifiquement sous la forme d'un système matriciel bioadhésif constitué d'un film d'épaisseur préférentiellement comprise entre 0,2 et 3,0 mm.

Ce type de système est particulièrement avantageux par rapport à un comprimé bioadhésif : en effet, il permet d'améliorer la tolérance par le patient en raison de la faible épaisseur du système ; d'autre part, il permet la libération accélérée de principes actifs peu solubles.

La forme galénique peut être de forme circulaire, rectangulaire ou oblongue et de surface préférentiellement comprise entre 0,1 et 5,0 cm².

Parmi les plastifiants utilisés, on peut citer le glycérol, la glycérine, le Transcutol®, le PEG 400, le propylène glycol, etc...

La forme galénique peut également contenir des excipients édulcorants tels que le saccharinate de sodium ainsi que des excipients aromatisants.

Lors de la fabrication, le mélange du composé (A), du composé (B), du ou des principes actifs, de l'excipient jouant le rôle de plastifiant et des autres excipients (par exemple un aromatisant) est distribué par étalement ou tout autre procédé sur un film protecteur biodégradable ou non ou sur un support (verre, inox...). L'ensemble est séché durant un temps compris entre 10 minutes et 2 heures à une température comprise entre 30 et 70°C.

Le film protecteur sur lequel est étalé la préparation peut être choisi pour ses propriétés adhésives ou bioadhésives.
Un cas particulier de forme galénique peut être réalisée de la manière suivante : le film protecteur qui recouvre la partie matricielle du patch buccal peut former autour de celle-ci une couronne adhésive telle que le décrit le schéma suivant :

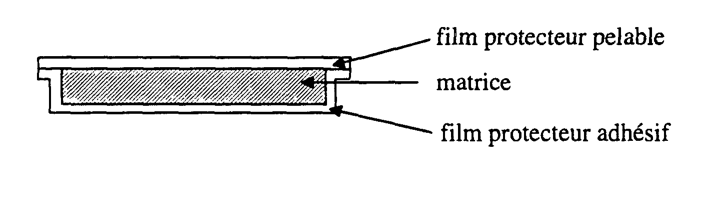

La composition pharmaceutique avec sa couronne adhésive présente l'avantage d'empêcher la sortie du principe actif par les côtés de la forme, ce qui, dans le cas de la muqueuse buccale par exemple, permet d'augmenter la fraction absorbée par voie transmuqueuse.

La composition pharmaceutique selon l'invention est caractérisée en ce qu'elle se maintient appliquée sur la muqueuse buccale, perlinguale, nasale, rectale ou vaginale pour une durée allant de 10 minutes jusqu'à 24 heures.

Parmi les principes actifs utilisés dans la composition pharmaceutique selon l'invention, on peut citer, à titre non limitatif : les antiinfectieux comme les pénicillines, les céphalosporines, les cyclines, les inhibiteurs de béta-lactamases, les aminosides, les quinolones, les nitroimidazolés, les sulfamides ou les antibactériens, les antihistaminiques, les antiallergiques, les anesthésiques, les antiinflammatoires stéroïdiens ou non, les antalgiques d'action locale ou systémique, les antispasmodiques, les anticancéreux, les diurétiques, les béta-bloquants, les antihypertenseurs, les antiangoreux, les antiarythmiques, les vasodilatateurs, les bradycardisants, les inhibiteurs calciques, les sédatifs, les cardiotoniques, les antifungiques, les antiulcéreux, les veinotoniques, les vasculoprotecteurs, les antiischémiques, les antiémétiques, les antispasmodiques, les anticoagulants, les antithrombotiques, les immunosuppresseurs, les immunomodulateurs, les antiviraux, les antidiabétiques, les hypolipidémiants, les antiobésités, les anticonvulsivants, les hypnotiques, les antiparkinsoniens, les antimigraineux, les neuroleptiques, les anxiolytiques, les antidépresseurs, les psychostimulants, les promnésiants, les bronchodilatateurs, les antitussifs, les antiostéoporotiques, les hormones peptidiques, les stéroïdes, les enzymes, les inhibiteurs d'enzymes, les agonistes ou antagonistes mélatoninergiques.

Les exemples suivants illustrent l'invention.

### EXEMPLE 1:

0,15 g de dihydroergotamine monométhanesulfonate sont mis en solution avec 5 g de copolymère d'acétate de vinyle/polyvinylpyrrolidone (de type PVP/VA E-735, ISP France) (en poids sec) dans 10 ml de solution alcool/acide chlorhydrique 0,1 N 50/50. Le copolymère de polyvinylpyrrolidone/acétate de vinyle de type E-735 est de composition suivante : 70 % de polyvinylpyrrolidone et 30 % d'acétate de vinyle. Lorsque le mélange est homogène, on ajoute 0,5 g de propylène glycol. On laisse agiter 30 minutes jusqu'à obtention d'un mélange parfaitement homogène.
Le mélange obtenu est étalé sur un film protecteur d'éthylène/acétate de vinyle. Le film est séché à température ambiante pendant 2 heures. Des disques de 1 cm de diamètre sont ensuite découpés à l'emporte-pièce. L'épaisseur des disques est d'environ 0,2 mm.

### EXEMPLE 2 :

0,88 g de dihydroergotamine monométhanesulfonate sont mis en solution avec 5 g de copolymère d'acétate de vinyle/polyvinylpyrrolidone (de type PVP/VA E-735, ISP France) dans 10 ml de solution alcool/acide chlorhydrique 0,1 N 50/50. Le copolymère d'acétate de vinyle/polyvinylpyrrolidone de type E-735 est de composition suivante : 70 % de polyvinylpyrrolidone et 30 % d'acétate de vinyle. Lorsque le mélange est homogène, on ajoute 0,5 g de propylène glycol. On laisse agiter 30 minutes jusqu'à obtention d'un mélange parfaitement homogène.
Le mélange obtenu est étalé sur un film protecteur d'éthylène/acétate de vinyle. Le film est séché à température ambiante pendant 2 heures. Des disques de 1 cm de diamètre sont ensuite découpés à l'emporte-pièce.

### EXEMPLE 3 :

0,7 g de dihydroergotamine mésylate sont mis en solution avec 4,5 g de copolymère d'acétate de vinyle/polyvinylpyrrolidone dans 4 ml de solution alcoolique. Le copolymère d'acétate de vinyle/polyvinylpyrrolidone de type VA 64 de chez BASF est de composition suivante : 60 % de polyvinylpyrrolidone et 40 % d'acétate de vinyle. Lorsque le mélange est homogène, on ajoute 0,15 g de saccharinate de sodium. Au mélange homogène, on ajoute ensuite 1,00 g de polyéthylène glycol 400 puis 0,4 g d'un mélange liquide d'aromatisants.
Le mélange obtenu est étalé sur un film protecteur d'éthylène/acétate de vinyle. Le film est séché à température ambiante pendant 2 heures. Des disques de 1 cm de diamètre sont ensuite découpés à l'emporte-pièce.

### EXEMPLE 4 :

1,0 g d'amineptine chlorhydrate est mis en solution avec 7,46 g de copolymère d'acétate de vinyle/polyvinylpyrrolidone dans 8,75 ml de solution alcoolique renfermant les conservateurs. Le copolymère d'acétate de vinyle/polyvinylpyrrolidone de type VA 64 de chez BASF est de composition suivante : 60 % de polyvinylpyrrolidone et 40 % d'acétate de vinyle. Lorsque le mélange est homogène, on ajoute 6,25 ml d'une solution aqueuse renfermant 0,24 g de saccharinate de sodium. Au mélange homogène obtenu, on ajoute ensuite 1,5 g de polyéthylène glycol 400 puis 0,55 g d'un mélange liquide d'aromatisants.
Le mélange obtenu est étalé sur un film protecteur d'éthylène/acétate de vinyle. Le film est séché à température ambiante pendant 2 heures. Des disques de 1 cm de diamètre sont ensuite découpés à l'emporte-pièce.

### EXEMPLE 5 :

1,0 g de piribédil monométhane sulfonate est mis en solution avec 7,31 g de copolymère d'acétate de vinyle/polyvinylpyrrolidone dans 8,75 ml de solution alcoolique renfermant les conservateurs. Le copolymère d'acétate de vinyle/polyvinylpyrrolidone de type VA 64 de chez BASF est de composition suivante : 60 % de polyvinylpyrrolidone et 40 % d'acétate de vinyle. Lorsque le mélange est homogène, on ajoute 6,25 ml d'une solution aqueuse renfermant 0,24 g de saccharinate de sodium. Au mélange obtenu, on ajoute ensuite 1,5 g de polyéthylène glycol 400 puis 0,55 g d'un mélange liquide d'aromatisants.
Le mélange obtenu est étalé sur un film protecteur d'éthylène/acétate de vinyle. Le film est séché à température ambiante pendant 2 heures. Des disques de 1 cm de diamètre sont ensuite découpés à l'emporte-pièce.

### EXEMPLE 6 :

0,25 g de mélatonine est mis en solution avec 7,46 g de copolymère d'acétate de vinyle/polyvinylpyrrolidone dans 8,75 ml de solution alcoolique renfermant les conservateurs. Le copolymère d'acétate de vinyle/polyvinylpyrrolidone de type VA 64 de chez BASF est de composition suivante : 60 % de polyvinylpyrrolidone et 40 % d'acétate de vinyle. Lorsque le mélange est homogène, on ajoute 6,25 ml d'une solution aqueuse renfermant 0,24 g de saccharinate de sodium. Au mélange obtenu, on ajoute ensuite 1,5 g de polyéthylène glycol 400, puis 0,55 g d'un mélange d'aromatisants.
Le mélange obtenu est étalé sur un film protecteur d'éthylène/acétate de vinyle. Le film est séché à température ambiante pendant 2 heures. Des disques de 1 cm de diamètre sont ensuite découpés à l'emporte-pièce.

### EXEMPLE 7 :

1,0 g de fusafungine est mis en solution avec 6,7 g de copolymère d'acétate de vinyle/polyvinylpyrrolidone dans 8,75 ml de solution alcoolique renfermant les conservateurs. Le copolymère d'acétate de vinyle/polyvinylpyrrolidone de type VA 64 de chez BASF est de composition suivante : 60 % de polyvinylpyrrolidone et 40 % d'acétate de vinyle. Lorsque le mélange est homogène, on ajoute 1 ml d'une solution aqueuse renfermant 0,24 g de saccharinate de sodium. Au mélange obtenu, on ajoute ensuite 1,50 g de polyéthylène glycol 400, puis 0,55 g d'un mélange d'aromatisants. Le mélange obtenu est étalé sur un film protecteur d'éthylène/acétate de vinyle. Le film est séché à température ambiante pendant 2 heures. Des disques de 1 cm de diamètre sont ensuite découpés à l'emporte-pièce.

### EXEMPLE 8 :

1,0 g de fusafungine est mis en solution avec 6,7 g de copolymère d'acétate de vinyle/polyvinylpyrrolidone dans 8,75 ml de solution alcoolique renfermant les conservateurs. Le copolymère d'acétate de vinyle/polyvinylpyrrolidone de type VA 64 de chez BASF est de composition suivante : 60 % de polyvinylpyrrolidone et 40 % d'acétate de vinyle. Lorsque le mélange est homogène, on ajoute 1 ml d'une solution aqueuse renfermant 0,24 g de saccharinate de sodium. Au mélange obtenu, on ajoute ensuite 1,50 g de polyéthylène glycol 400, puis 0,47 g d'une composition aromatique. Le mélange obtenu est étalé sur un film protecteur d'éthylène/acétate de vinyle. Le film est séché à température ambiante pendant 2 heures. Des disques de 1 cm de diamètre sont ensuite découpés à l'emporte-pièce.

### EXEMPLE 9 :

1,0 g de fusafungine et 1,0 g de chlorhydrate de lidocaïne sont mis en solution avec 6 g de copolymère d'acétate de vinyle/polyvinylpyrrolidione dans 8,75 ml de solution alcoolique renfermant les conservateurs. Le copolymère d'acétate de vinyle/polyvinylpyrrolidone de type VA 64 de chez BASF est de composition suivante : 60 % de polyvinylpyrrolidone et 40 % d'acétate de vinyle. Lorsque le mélange est homogène, on ajoute 1 ml d'une solution aqueuse renfermant 0,23 g de saccharinate de sodium. Au mélange obtenu, on ajoute ensuite 1,30 g de polyéthylène glycol 400, puis 0,47 g d'une composition aromatique. Le mélange obtenu est étalé sur un film protecteur d'éthylène/acétate de vinyle. Le film est séché à température ambiante pendant 2 heures. Des disques de 1 cm de diamètre sont ensuite découpés à l'emporte-pièce.

### EXEMPLE 10 :

1,0 g de fusafungine et 0,4 g de chlorhydrate de lidocaïne sont mis en solution avec 6,6 g de copolymère d'acétate de vinyle/polyvinylpyrrolidone dans 8,75 ml de solution alcoolique renfermant les conservateurs. Le copolymère d'acétate de vinyle/polyvinylpyrrolidone de type VA 64 de chez BASF est de composition suivante : 60 % de polvinylpyrrolidone et 40 % d'acétate de vinyle. Lorsque le mélange est homogène, on ajoute 1 ml d'une solution aqueuse renfermant 0,23 g de saccharinate de sodium. Au mélange obtenu, on ajoute ensuite 1,30 g de polyéthylène glycol 400, puis 0,47 g d'une composition aromatique. Le mélange obtenu est étalé sur un film protecteur d'éthylène/acétate de vinyle. Le film est séché à température ambiante pendant 2 heures. Des disques de 1 cm de diamètre sont ensuite découpés à l'emporte-pièce.

### EXEMPLE 11 :

0,05 g de bétamethasone 17-valérate est mis en solution avec 7,66 g de copolymère d'acétate de vinyle/polyvinylpyrrolidone dans 8,75 ml de solution alcoolique renfermant les conservateurs. Le copolymère d'acétate de vinyle/polyvinylpyrrolidone de type VA 64 de chez BASF est de composition suivante : 60 % de polyvinylpyrrolidone et 40 % d'acétate de vinyle. Lorsque le mélange est homogène, on ajoute 6,25 ml d'une solution aqueuse renfermant 0,24 g de saccharinate de sodium. Au mélange obtenu, on ajoute ensuite 1,50 g de polyéthylène glycol 400, puis 0,55 g d'un mélange d'aromatisants. Le mélange obtenu est étalé sur un film protecteur d'éthylène/acétate de vinyle. Le film est séché à température ambiante pendant 2 heures. Des disques de 1 cm de diamètre sont ensuite découpés à l'emporte-pièce.

### EXEMPLE 12 :

0,11 g de chlorure de cétylpyridinium est mis en solution avec 7,6 g de copolymère d'acétate de vinyle/polyvinylpyrrolidone dans 8,75 ml de solution alcoolique renfermant les conservateurs. Le copolymère d'acétate de vinyle/polyvinylpyrrolidone de type VA 64 de chez BASF est de composition suivante : 60 % de polyvinylpyrrolidone et 40 % d'acétate de vinyle. Lorsque le mélange est homogène, on ajoute 6,25 ml d'une solution aqueuse renfermant 0,24 g de saccharinate de sodium. Au mélange obtenu, on ajoute ensuite 1,50 g de polyéthylène glycol 400, puis 0,55 g d'un mélange d'aromatisants. Le mélange obtenu est étalé sur un film protecteur d'éthylène/acétate de vinyle. Le film est séché à température ambiante pendant 2 heures. Des disques de 1 cm de diamètre sont ensuite découpés à l'emporte-pièce.

## Revendications

1. Composition pharmaceutique bioadhésive pour la libération contrôlée de principes actifs dans la cavité buccale ou à travers une muqueuse **caractérisée en ce que** la bioadhésion et la libération contrôlée sont assurées par un composé (A) consistant en un ou plusieurs copolymères acétate de vinyle / polyvinylpyrrolidone, la quantité du composé (A) étant comprise entre 5 et 85 % de la masse totale de la composition.

2. Composition pharmaceutique selon la revendication 1 **caractérisée en ce qu'**elle contient, outre le composé (A), un ou plusieurs principes actifs et un ou plusieurs excipients thérapeutiquement acceptables.

3. Composition pharmaceutique selon la revendication 1 **caractérisée en ce qu'**elle contient, outre le composé (A), un ou plusieurs principes actifs, un ou plusieurs excipients pharmaceutiquement acceptables et un composé (B) comprenant un ou plusieurs composés choisi parmi la cellulose et ses dérivés, les amidons et leurs dérivés, et les carraghénates.

4. Composition pharmaceutique selon l'une quelconque des revendications 2 et 3 **caractérisée en ce que** les excipients pharmaceutiquement acceptables sont choisis parmi les plastifiants, les aromatisants et les édulcorants.

5. Composition pharmaceutique selon la revendication 3 **caractérisée en ce que** la quantité du composé (B) est comprise entre 5 et 85 % de la masse totale de la composition.

6. Composition pharmaceutique selon la revendication 1 **caractérisée en ce qu'**elle se présente sous la forme d'un système matriciel.

7. Composition pharmaceutique selon l'une quelconque des revendications 1, 2 et 3 **caractérisée en ce que** la bioadhésion est assurée pour une période allant jusqu'à 24 heures.

8. Composition pharmaceutique selon l'une quelconque des revendications 2 et 3 **caractérisée en ce que** le ou les principes actifs utilisés sont choisis parmi les antiinfectieux comme les pénicillines, les céphalosporines, les cyclines, les inhibiteurs de béta-lactamases, les aminosides, les quinolones, les nitroimidazolés, les sulfamides ou les antibactériens, les antihistaminiques, les antiallergiques, les anesthésiques, les antiinflammatoires stéroïdiens ou non, les antalgiques d'action locale ou systémique, les antispasmodiques, les anticancéreux, les diurétiques, les béta-bloquants, les antihypertenseurs, les antiangoreux, les antiarythmiques, les vasodilatateurs, les bradycardisants, les inhibiteurs calciques, les sédatifs, les cardiotoniques, les antifungiques, les antiulcéreux, les veinotoniques, les vasculoprotecteurs, les antiischémiques, les antiémétiques, les antispasmodiques, les anticoagulants, les antithrombotiques, les immunosuppresseurs, les immunomodulateurs, les antiviraux, les antidiabétiques, les hypolipidémiants, les antiobésités, les anticonvulsivants, les hypnotiques, les antiparkinsoniens, les antimigraineux, les neuroleptiques, les anxiolytiques, les antidépresseurs, les psychostimulants, les promnésiants, les bronchodilatateurs, les antitussifs, les antiostéoporotiques, les hormones peptidiques, les stéroïdes, les enzymes, les inhibiteurs d'enzymes, les agonistes ou antagonistes mélatoninergiques.

## Claims

1. Bioadhesive pharmaceutical composition for the controlled release of active ingredients in the buccal cavity or through a mucous membrane, **characterised in that** the bioadhesion and controlled release are ensured by a component (A) consisting of one or more vinyl acetate/polyvinylpyrrolidone copolymers, the amount of component (A) being from 5 to 85 % of the total weight of the composition.

2. Pharmaceutical composition according to claim 1, **characterised in that** it comprises, besides component (A), one or more active ingredients and one or more therapeutically acceptable excipients.

3. Pharmaceutical composition according to claim 1, **characterised in that** it comprises, besides component (A), one or more active ingredients, one or more pharmaceutically acceptable excipients and a component (B) comprising one or more compounds selected from cellulose and derivatives thereof, starches and derivatives thereof, and carragheenates.

4. Pharmaceutical composition according to any one of claims 2 and 3, **characterised in that** the pharmaceutically acceptable excipients are selected from plasticisers, flavourings and sweeteners.

5. Pharmaceutical composition according to claim 3, **characterised in that** the amount of component (B) is from 5 to 85 % of the total weight of the composition.

6. Pharmaceutical composition according to claim 1, **characterised in that** it is presented in the form of a matrix system.

7. Pharmaceutical composition according to any one of claims 1, 2 and 3, **characterised in that** the bioadhesion is ensured for a period of up to 24 hours.

8. Pharmaceutical composition according to any one of claims 2 and 3, **characterised in that** the active ingredient(s) used are selected from anti-infective agents such as penicillins, cephalosporins, cyclines, beta-lactamase inhibitors, aminosides, quinolones, nitroimidazole compounds, sulphamides or antibacterials, antihistamines, anti-allergics, anaesthetics, steroidal or non-steroidal anti-inflammatories, antalgics having local or systemic action, antispasmodics, anti-cancer agents, diuretics, beta-blockers, antihypertensives, anti-angina agents, anti-arrythmics, vasodilators, bradycardiacs, calcium inhibitors, sedatives, cardiotonics, antifungals, anti-ulcerative agents, venotonics, vasculoprotectors, anti-ischaemics, anti-emetics, antispasmodics, anticoagulants, antithrombotics, immuno-suppressors, immunomodulators, antivirals, antidiabetics, hypolipidaemic agents, anti-obesity agents, anticonvulsants, hypnotics, antiparkinsonian agents, antimigraine agents, neuroleptics, anxiolytics, antidepressants, psychostimulants, memory-enhancers, bronchodilators, antitussives, anti-osteoporotics, peptide hormones, steroids, enzymes, enzyme inhibitors, and melatoninergic agonists and antagonists.

## Patentansprüche

1. Bioadhesive pharmazeutische Zubereitung zur gesteuerten Freisetzung von Wirkstoffen in der Mundhöhle oder über eine Schleimhaut, **dadurch gekennzeichnet, daß** die Bioadhesivität und die gesteuerte Freisetzung durch eine Verbindung (A), die aus einem oder mehreren Vinylacetat/Polyvinylpyrrolidon-Copolymeren besteht, sichergestellt werden, wobei die Menge der Verbindung (A) zwischen 5 und 85 % der Gesamtmasse der Zubereitung beträgt.

2. Pharmazeutische Zubereitung nach Anspruch 1, **dadurch gekennzeichnet, daß** sie neben der Verbindung (A) einen oder mehrere Wirkstoffe und einen oder mehrere therapeutisch verträgliche Trägermaterialien enthält.

3. Pharmazeutische Zubereitung nach Anspruch 1, **dadurch gekennzeichnet, daß** sie neben der Verbindung (A) einen oder mehrere Wirkstoffe, einen oder mehrere pharmazeutisch annehmbare Trägermaterialien und eine Verbindung (B) enthält, die eine oder mehrere Verbindungen ausgewählt aus Cellulose und ihren Derivaten, Stärken und deren Derivaten und Carrageenaten umfaßt.

4. Pharmazeutische Zubereitung nach einem der Ansprüche 2 und 3, **dadurch gekennzeichnet, daß** die pharmazeutisch annehmbaren Trägermaterialien aus Weichmachern. Aromastoffen und Süßungsmitteln ausgewählt sind.

5. Pharmazeutische Zubereitung nach Anspruch 3, **dadurch gekennzeichnet, daß** die Menge der Verbindung (B) zwischen 5 und 85 % der Gesamtmasse der Zubereitung beträgt.

6. Pharmazeutische Zubereitung nach Anspruch 1, **dadurch gekennzeichnet. daß** sie in Form eines Matrix-Systems vorliegt.

7. Pharmazeutische Zubereitung nach einem der Ansprüche 1, 2 und 3, **dadurch gekennzeichnet, daß** die Bioadhesivität für eine Zeitdauer von bis zu 24 Stunden sichergestellt ist.

8. Pharmazeutische Zubereitung nach einem der Ansprüche 2 und 3, **dadurch gekennzeichnet, daß** der oder die verwendeten Wirkstoffe ausgewählt sind aus antiinfektiösen Mitteln, wie Penicillinen, Cephalosporinen, Cyclinen, Beta-Lactamase-Inhibitoren, Aminosiden, Chinolonen, Nitroimidazolen, Sulfamiden oder antibakteriellen Mitteln, Antihistaminika, Antiallergika, Anästhetika, Steroid- oder Nichtsteroid-Antiinflammatorika, Analgetika mit lokaler oder systemischer Wirkung, antispasmodischen Mitteln, Antikrebsmitteln, Diuretika, Beta-Blockern, antihypertensiven Mitteln, Antiangoramitteln, Antiarhythmika, vasodllatatorischen Mitteln, bradycardisierenden Mitteln, Calcium-Inhibitoren, Sedativa, Cardiotonika, antifungistischen Mitteln, Anti-Geschwürmitteln, venentoxischen Mitteln, gefäßschützenden Mitteln, Antiischämlka, Antiemetika, Antispasmodika. Anticoagulantien, antithromobotischen Mitteln, immunosuppressiven Mitteln, immunomodulierenden Mitteln, antiviralen Mitteln, Antidiabetika, hypolipidämischen Mitteln, gegen die Fettsucht wirkenden Mitteln, anticonvulsiven Mitteln, Hypnotika, Anti-Parkinson-Mitteln, Anti-Migräne-Mitteln, Neuroleptika, Anxiolytika, Antidepressiva, Psychostimulantien, promnesischen Mitteln, Bronchodilatatoren, Anti-Husten-Mitteln, gegen die Osteoporose gerichteten Mitteln. Peptidhormonen, Steroiden, Enzymen, Enzym-Inhibitoren und melatoninergischen Agonisten oder Antagonisten.
